# EUROPEAN PATENT SPECIFICATION

(11) **EP 0 834 562 B1**
(45) Date of publication and mention of the grant of the patent: **05.11.2003**
(21) Application number: 97102577.0
(22) Date of filing: 22.02.1990
(51) Int. Cl.: C12N 15/12, C12N 15/62, C07K 14/81

(54) **Polypetide exhibiting calpain inhibition activity and method for the preparation thereof**
Polypeptide mit Calpaininhibitionswirkung und Verfahren zu deren Herstellung
Polypeptides exhibant une activité d'inhibition de la calpaine et leur procédé de préparation

(30) Priority: 03.03.1989 JP 4978489; 14.11.1989 JP 29403389
(43) Date of publication of application: 08.04.1998
(62) Divisional of application: 90301912.3
(73) Proprietor: FUJITA HEALTH UNIVERSITY, Toyoake-shi, Aichi-ken (JP); TAKARA BIO INC., Otsu-shi, Shiga (JP)
(72) Inventor: Sekiguchi, Kiyothoshi, Sakai-shi Osaka-fu (JP); Maeda, Toshinaga, Kusatsu-shi Shiga-ken (JP); Titani, Koiti, Kasugai-shi, Aichi-ken (JP); Kimizuka, Fusao, Ohmihachiman-shi, Shiga-ken (JP); Kato, Ikunoshin, Uji-shi, Kyoto-fu (JP)
(74) Representative: Marlow, Nicholas Simon

(56) References cited:
- EP-A- 0 207 751
- EP-A- 0 291 804
- WO-A-86/00090
- MURACHI T: "Intracellular regulatory system involving calpain and calpastatin." BIOCHEM INT, FEB 1989, 18 (2) P263-94, AUSTRALIA, XP002046602
- TAKANO E ET AL: "Evidence for the repetitive domain structure of pig calpastatin as demonstrated by cloning of complementary DNA." FEBS LETT, NOV 24 1986, 208 (2) P199-202, NETHERLANDS, XP002046601

## Description

This invention is related to a polypeptide exhibiting calpain inhibition activity, more particularly to such a polypeptide to which cell adhesive activity has been introduced.

With the recent advances in molecular biology, the mechanisms by which cells and the extracellular matrix adhere are coming to be understood on the molecular level. Or the extracellular matrix proteins, Fibronectin (FN) was the first round to contain an essential sequence for cell adhesion. Thus the Arg-Gly-Asp-Ser sequence (RGDS) in the cell-binding domain or FN has been round to be essential for cell adhesion by Ruoslahti et al. (Nature, 309, 30-33, 1984). The RGD part of this sequence is needed for cell adhesion and substitution with other amino acids cannot be done without loss or cell adhesive activity, but the serine can be replaced by, for example, threonine, alanine, cysteine, or valine without loss of activity. However, if substitution is with proline or lysine, the activity is lost. Proteins other than FN which contain the sequence RGD include thrombin, vitronectin, von Willebrand factor, ribrinogen, collagen, discoidin I, λ-phage receptor, and others. It has thus been suggested that the RGD sequence is closely related to protein functions (Ruoslahti et al., Proc. Natl. Acad. Sci. USA, 81, 5985-5988, 1984). However, it is not certain whether the RGD sequence in these molecules confers cell adhesive activity. For example, although fibrinogen has the RGDS sequence, it does not have cell adhesive effects on fibroblasts.

Another example of cell adhesive protein in addition to those named above is laminin. Laminin is a glycoprotein or high molecular weight found in the basement membrane, and it has cell adhesive activity toward a variety of cells in the epithelium. It has been reported (Graf et al., Cell, 48, 989-996, 1987) that the smallest sequence related to cell adhesion is Tyr-Ile-Gly-Ser-Arg (YIGSR). Laminin also has the RGD sequence, but it is not known if the sequence is related to the cell adhesive activity.

In addition, it is known that the Glu-Ile-Leu-Asp-Val (EILDV) sequence in the IIICS domain of FN is related to the adhesion of lymph cells and melanoma cells.

A technique by which a synthetic polypeptide can be bound to a polypeptide originating from FN at the N-terminal of tumor necrosis factor (TNF) has been disclosed (Japanese Laid-Open Patent Application No. 297399/88). The polypeptide bound with TNF is a portion of the sequence or the cell-binding domain of FN, and it contains the sequence RGDS. The synthetic polypeptide obtained as a result of the use or the technique has stronger activity in causing tumor necrosis than TNF. However, in this technique, two functional polypeptides are bound by the use of gene engineering, and the results do not show that the RGDS sequence has the cell adhesive activity within the TNF molecule.

If it were possible to confer cell adhesive activity on polypeptide exhibiting calpain inhibiting activity, it would be possible to increase the affinity between cells and the polypeptide. If this is possible, the technique would be very useful in the development of new drug delivery systems, sustained-release drugs, and so on.

The object of this invention is to provide a technique by which cell adhesive activity can be conferred on polypeptides exhibiting calpain inhibition activity without changing their original function.

The present invention relates to an artificial polypeptide exhibiting calpain inhibition activity, to the molecule of which has been introduced a peptide which has cell adhesive activity. This invention also relates to a technique based on gene engineering for the introduction of DNA which codes for a peptide which has cell adhesive activity to DNA which codes for the polypeptide, and the use of a plasmid vector which carries said DNA to transform host cells so that they then express the polypeptide.

The inventors of this invention have accomplished this invention by the introduction of a peptide which has cell adhesive activity into an appropriate region of the polypeptide which does not have cell adhesive activity, thus producing a functional polypeptide having cell adhesive activity.

The inventors inserted the sequence RGDS into the active fragment of calpastatin as follows.

The DNA sequence which corresponds to RGDS was inserted so as to match the reading frame into the coding region of a plasmid which expresses a fragment which includes domain I of human calpastatin, and the resulting DNA was introduced into E. coli. The biological activity of the polypeptide expressed by the recombinants was evaluated. Both calpastatin activity and cell adhesive activity were found. Calpastatin is an endogenous protease inhibitor which specifically inhibits calpain (EC 3.4.22.17), a Ca-dependent neutral protease. Calpastatin may be useful in the treatment of a variety of disorders caused by excess calpain activity or decreased calpastatin activity, such as hypertension, muscular dystrophy, cataracts, etc. If cell adhesive activity is conferred on calpastatin, its pharmacological effects may be enhanced.

The above example of the insertion of the sequence RGDS into the polypeptide shows that it is now possible to produce the functional polypeptides which has both the calpain inhibition function of the polypeptide before modification and cell adhesive activity as well.

This invention will be explained in detail as follows.

It is preferable that the sequence to be inserted into the polypeptide contains, at the least, the sequence RGD. The amino acid immediately to the C-terminal side of the RGD sequence can be any amino acid other than lysine (K), proline (P), or hydroxyproline (HyP). There seems to be no one-letter code name. There is no particular restriction as to the amino acid immediately to the N-terminal side of the sequence RGD. The insertion site can be any site preferred in the polypeptide, but it is best that the site be one at which such insertion does not cause loss of the original calpain inhibition function of the polypeptide. Also, it is preferred that the sequence RGD be inserted in a site at which the tertiary structure can be readily recognised by cell receptors. It is necessary that the gene for the polypeptide into which the sequence RGD is to be inserted is carried by a plasmid which can be expressed when introduced into the host. The DNA sequence corresponding to the sequence RGD is inserted into the coding region of the plasmid so that the reading frame matches, and by the expression of the polypeptide by the host, the polypeptide which contains the RGD sequence can be produced.

The insertion of the sequence RGD into calpastatin will be explained in more detail.

Calpastatin is a single-chain protein that contains four domains, I-IV, consisting of 120 to 140 amino acids each, and a domain L at the N-terminal side. Domains I-IV each independently inhibits calpain, but domain L is inactive. The cDNA of human calpastatin was cloned, and various fragments were inserted into expression vectors; then, an expression system was established in E. coli. In this invention, expression plasmid pHCSd42, which expresses a peptide which consisted of 133 amino acids (called HCSd42 here) containing most or domain I of human calpastatin and a part of domain L on its N-terminal side, and expression plasmid pHCSd421, which expressed a peptide (HCSd421) that consisted of 96 amino acids, being without the C-terminal 37 amino acids of HCSd42, were used as examples. These plasmids were constructed by the steps described below and in Japanese Laid-Open Patent Application No. 283300/89 and Japanese Patent Application No. 126648/89.

First, two cDNA clones of human calpastatin, λcs131 and λcs19, were linked to yield a cDNA that codes for most of domain L and the entire region of domains I-IV. The cDNA obtained was then used to construct an expression plasmtd pHCS121 by linkage at the NcoI-EcoRI site of an expression vector, pUC119N. Cells of the strain carrying this plasmid, E. coli JM109/pHCS121, were deposited at the Fermentation Research Institute of the Agency of Industrial Science and Technology as FERM P-9989 (Japanese Laid-Open Application No. 283300/90).

A transcription terminator was inserted in the EcoRI-SalI site of this plasmid pHCS121, and the region corresponding to domains II-IV was deleted with the use of SacI-XbaI, giving plasmid pHCS82. Restriction sites AvaIII and ClaI were introduced into the region corresponding to domain L, giving pHCS83. Then stop codon was inserted into the site corresponding to domain I at the C-terminal, giving pHCS84. The AvaIII-SalI fragment or pHCS84 was linked with the PstI-SalI site of pTV119N, and a large portion of the 5'-end of the region corresponding to domain L was removed with the use of nuclease. The plasmid obtained was closed again at the NcoI site, giving pHCSd42. Cells of E. coli545π HR/pHCSd42, which carry this plasmid, were deposited at the Fermentation Research Institute of the Agency of Industrial Science and Technology as FERM P-10649. Another stop codon was inserted upstream of the stop codon or pHCSd42, giving pHCSd421 (Japanese Patent Application No. 126648/89).

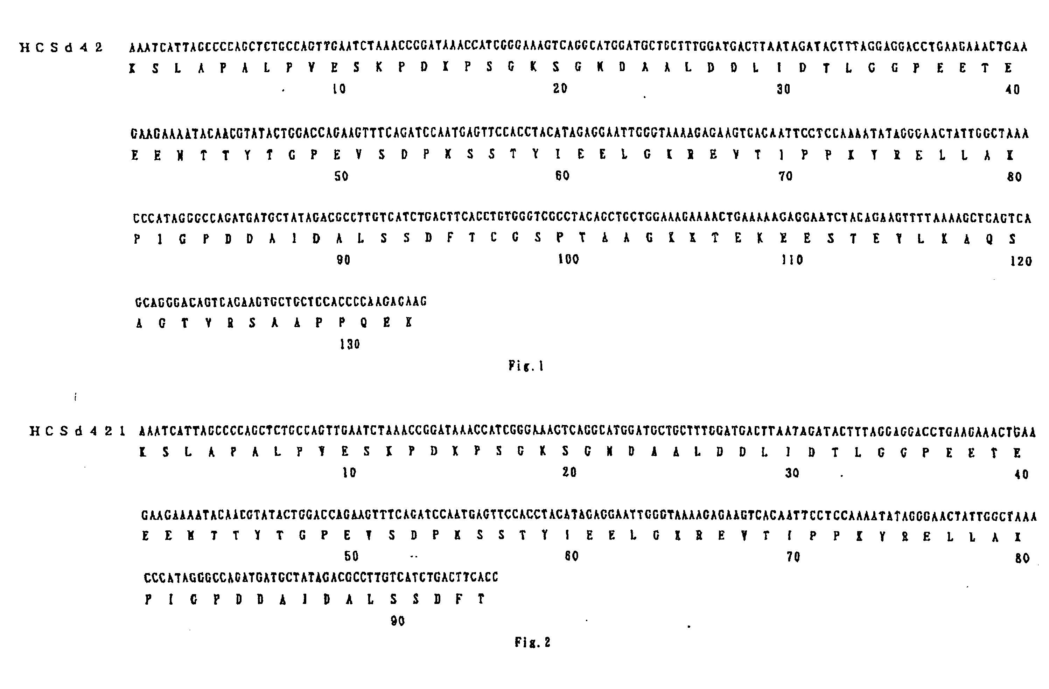

The amino acid sequence and the DNA sequence of HCSd42 are shown in Figure 1 and those of HCSd421 are shown in Figure 2. The site or the insertion of the RGD sequence should be near the surface or the molecule, and should be in a site not essential to the expression of calpastatin activity. When the three-dimensional structure of the molecule is not known, models such as, for example, the Chou-Fasman model for the prediction of secondary structure from the primary structure should be considered. Based on these considerations, appropriate sites for insertion can be chosen. Site-directed mutagenesis is an effective procedure for use in the insertion. Because pHCSd42 and pHCSd421 are from pTV119N as a vector, which can produce single-stranded DNA it is easy for single-stranded DNA for use as a template to be prepared. That is, a plasmid is caused to replicate in the presence of deoxyuridine (dU) in a host that cannot decompose dU, and by infection with helper phage, single-stranded DNA that incorporated dU is produced, which is purified and used as a template. Separately, DNA that contains the complementary DNA sequence to the base sequences at the Insertion sites to the 5' end and the 3' end of the DNA sequence which corresponds to the RGD sequence (the complementary sequences are each 8-10 bases long) is synthesized and used as primer in the synthesis of double-stranded DNA in the presence of DNA polymerase. This DNA is used to transrorm host cells which can decompose dU. Then the desired clone is selected from the transformants obtained; so that selection will be easy, the primer should be designed in the appropriate way so that a restriction site will be generated by of the insertion of the DNA sequence that corresponds to RGD, and so that already existing sites will disappear.

The transformants obtained in this way are cultured, and the expression or the desired polypeptide is induced. Next, the bacterial cells are disrupted, and the disrupted cells are heated at 100°C for about 10 minutes, after which the suspension is centrifuged and the supernatant is tested for calpastatin activity. The assay of calpastatin can be by the method of Murakami et al. (J. Biochem., 90, 1809-1816, 1981). If expression of the polypeptide with calpastatin activity is found, the desired product can be readily purified by the treatment of the supernatant by chromatography, such as, for example, DEAE ion-exchange chromatography. The cell adhesive activity of the polypeptide obtained can be evaluated by the method described above. By this procedure, it is possible to obtain a new polypeptide with both calpastatin activity and cell adhesive activity.

The insertion of the RGD sequence which has cell adhesive activity into a polypeptide has been explained. However, it is also possible for the same method to be used for the insertion of a sequence other than the RGD sequence into a polypeptide, by which the multifunctional polypeptide of this invention can be obtained.

This invention will be further explained by reference to examples, but this invention is not to be taken to be limited to these examples.

### Example 1

Preparation of a calpastatin-like polypeptide, HCSd342, containing the RGDS sequence:

### (1-1). Construction of the plasmid.

Single-stranded DNA in which some or the thymidines were replaced by dU was prepared From plasmid pHCSd42, which expresses HCSd42, by the use of a commercially available kit for mutagenesis, Mutan K (Takara Shuzo). The procedure recommended by the manufacturer was followed. Then, pHCSd42 was used to transrorm E. coli BW313 (dut⁻, ung⁻), and the transformed cells were cultured for about 5 hours on 2 x YT medium. Then 1% inoculation was made of new medium (5 ml x 10 tubes). At the same time, the cells were infected with helper phage M13K07, and left for 30 minutes at 37°C. Then culture was continued overnight in the presence of 70 µg/ml kanamycin. To the culture supernatant, a one-fourth volume of 20% PEG containing 2.5 M NaCl was added and mixed with agitation, after which the mixture was left for 30 minutes at 4°C, and the precipitate obtained. The precipitate was dissolved in 1 ml of TE, and treated with phenol. Ethanol precipitation was done twice, and the precipitate was dissolved in 30 µl of water. The yield was about 90 µg. Separately, the following DNA sequence (or 33 bases) was synthesized for use as a primer to induce mutagenesis for the insertion of the RGD sequence between the K⁸⁰ and the P⁸¹ of the amino acid sequence or HCSd42.

L A K R G D S G
5' TTG GCT AAA CGT GGT GAC TCC GGA
←[ I ]→ ←[ II ]→
P I G
CCC ATA GGG
←[ III ]→

Here, the sequences [I] and [III] correspond to the + chain of pHCSd42, and the sequence [II] is that sequence which codes for the RGDSG sequence. The G on the C-terminal side of RGDSG was added so that the mutant plasmid could be readily selected, and it is possible to recognize the desired clone by the construction or a unique AccIII site. The primer was synthesized on a DNA synthesizer (Applied Biosystems), and then purified by HPLC. Then 0.2 µg (20 pmol) of the 5' end or the DNA was made into a primer for use in mutagenesis by phosphorylation in the presence of polynucleotide kinase and ATP. As template, 0.04-1 pmol (4 µl) of the single-stranded DNA which contains dU prepared before was used; this was annealed with 4 pmol (4 µl) of primer in an annealing buffer. Then 2 µl or the annealed product was mixed with 25 µl of extension buffer, 1 µl or DNA ligase from E. coli, and 1 µl or T4DNA polymerase, and the mixture was incubated for 2 hours at 25°C, to allow synthesis of double-stranded chains. After 3 µl of 0.2 M EDTA was added to the reaction mixture, the reaction was stopped by heating or the reaction mixture for 5 minutes at 65°C, and 3 µl of the reaction mixture was used to transform E. coli BMH71-18mutS. The transformants obtained (12 clones) were cultured on LB broth, and their plasmid DNA was extracted and cleaved with SalI and then with AccIII, giving one clone that had fragments of 3.38 kb and 1.24 kb. This plasmid was named pHCSd342, and the base sequence of the DNA inserted into it was analyzed and found to be the desired mutation.

### (1-2). Production by E. coli of a calpastatin-like polypeptide in which the RGDSG sequence was inserted.

The plasmid pHCSd342 obtained in section (1-1) was used to transform cells of E. coli JM109 in the usual way. The transformants were named E. coli JM109/pHCSd342, and deposited as FERMBP-2719 at the Fermentation Research Institute of the Agency or Industrial Science and Technology.

Transformants were cultured with shaking at 37°C in 1 l of L-medium; when growth reached the logarithmic stage, IPTG was added to the final concentration or 2 mM, and culture was continued for the next 15 hours. After culture, the cells were cooled to 0°C, and NaN₃ was added to the final concentration of 0.02% and mixed in before the cells were harvested. The supernatant was discarded and the cells were washed with 40 ml of N buffer (20 mM MES(2-(N-morpholino)ethanesulfonic acid (pH 5.5)), containing 1 mM EDTA, 4 µM PMSF, and 5 mM 2-mercaptoethanol) and centrifuged. The cell pellet was suspended in 40 ml of N buffer, to which suspension was added lysozyme to the final concentration of 0.5 mg/ml, and the suspension was incubated at 0°C for 10 minutes. Next, the cells were disrupted with ultrasonic waves, and the suspension was centrifuged for 20 minutes at 16,000 x g. The supernatant was heated at 90°C for 10 minutes. The supernatant was centrifuged for 20 minutes at 12,000 x g, and this supernatant collected. This heat-treated supernatant was put through a eighty milliliters of DEAE-Toyopearl 650M column (Tosoh Corp.) equilibrated with 50 mM NaCl buffer, pH 5.5, that contained 20 mM MBS, 1 mM EDTA, and 4 µM PMSF.

The column was first washed with 350 ml of the above buffer, and then elution was done with 1000 ml of the same buffer containing NaCl in a linear concentration gradient of 50 to 200 mM. The calpastatin activity or the fractions obtained was measured by measurement or their inhibition of calpain (Kitahara et al., J. Biochem. 95, 1759, 1984). The sample was mixed with 2 µl (0.4 U) of calpain, 40 µl of 2% casein, and 6 mg/ml cysteine, and water was added to bring the volume to 180 µl. The mixture was heated at 30°C for 20 minutes. Then 20 µl of 50 mM calcium chloride was added, and the mixture was incubated For 20 minutes at 30°C. Next, 200 µl of 5% TCA was added to stop the reaction, and the reaction mixture was centrifuged for 10 minutes at 10,000 x g. The supernatant was collected and its absorbance at 280 nm was measured. From this result, the inhibition of calpain was calculated. The fractions that inhibited calpain were analyzed by SDS-polyacrylamide gel electrophoresis, and purified fractions were dialyzed against water and lyophilized. In this way, about 1 mg of product was obtained. This product was a calpastatin-like polypeptide that had the RGDSG sequence inserted between the K⁸⁰ and P⁸¹ of the HCSd42 polypeptide. It was named HCSd342.

### Example 2

Production of a calpastatin-like polypeptide HCSd4210 into which the RGDS sequence was inserted:

To insert the RGDS sequence between the G³⁵ and the P³⁶ of the calpastatin-like polypeptide HCSd42 that was encoded by pHCSd42, the following DNA was synthesized for use in the induction of mutation. 
T L G G R G D S
5' ACT TTA GGC* GGC* CGC GGT GAC TCT
P E E
CCT GAA GAA
 The underlined portion is the DNA which corresponds to the RGDS sequence to be inserted. The bases marked with asterisks form a NotI site, so although the plasmid sequence of the original plasmid is changed, the corresponding amino acids are not changed.

The same methods as in Example 1 were used to obtain a mutant plasmid pHCSd4210 that expressed HCSd4210 with the NotI site as the index. Next, E. coli JM109/pHCSd4210, which carried this plasmid, was cultured, and the calpastatin-like polypeptide HCSd4210 that contained the RGDS sequence was obtained.

### Example 3

Production of the calpastatin-like polypeptide HCSd427 into which the RGDS sequence had been inserted:

To insert the RGDS sequence between the S¹²⁰ and the A¹²¹ of the calpastatin-like polypeptide HCSd42 coded for by pHCSd42, the following DNA was synthesized for use in the induction of mutation. 
    A    Q    S     R    G    D    S    A
5'  GCT  CAG  TCG*  CGA  GGT  GAC  TCT  GCA

G    T
GGG  ACA
 The underlined portion is the DNA which corresponds to the RODS sequence to be inserted. The bases marked with asterisks form an NruI site, so although the sequence of the original plasmid is changed, the corresponding amino acid is not changed.

The same methods as in Example 1 were used to obtain a mutant plasmid pHCSd427 that expressed HCSd427 with the NruI site as the index. Next, E. coli JM109/pHCSd427, which carried this plasmid, was cultured, and the calpastatin-like polypeptide HCSd427 that contained the RGDS sequence was obtaiend.

### Example 4

Production of the calpastatin-like polypeptide HCSd4211 into which the RGDSG sequence had been inserted:

Starting with pHCSd42, the deletion mutant pHCSd421 was produced. The method of preparation was disclosed in Japanese Patent Application No. 126648/89. pHCSd421 codes for the calpastatin-like polypeptide coded for by pHCSd42, with the 37 amino acids on the C-terminal side omitted, leaving 96 amino acids in the polypeptide. Single-stranded DNA was prepared from pHCSd421 by the method used in Example 1. Next, primer with the same sequence as the primer used in mutagenesis in Example I was used to insert the RGDSG sequence between K⁸⁰ and P⁸¹. The procedure was exactly the same as in Example 1. Plasmid pHCSd4211, which expressed the calpastatin-like polypeptide HCSd4211 into which the RGDSG sequence had been inserted, was obtained. This plasmid pHCSd4211 was used to transform cells of E. coli JM109 in the usual way. The transformed cells were named E. coli JM109/pHCSd4211, and deposited as FERMBP-2720 at the Fermentation Research Institute of the Agency of Industrial Science and Technology. The transformants were cultured by the methods of Example 1, and from a cell extract, HCSd4211 was purified.

### Example 5

Measurement of cell adhesive activity of the calpastatin-like polypeptides.

The cell adhesive activity of the polypeptides which contained the RGDS sequence obtained in Example 1-4 was measured with the use of BHK cells.

### 1. Preparation of samples.

The samples were dissolved in 0.1 M NaHCO₃, and 400 µl of the dissolved sample was put into each well or a 24-well flat-bottomed plate (Terumo) and left overnight at 4°C to adsorb. The wells were washed with PBS, and then 500 µl of PBS containing 1% BSA was added to each well. The plate was incubated at room temperature for 3-4 hours, and the wells were washed with PBS before being used in the assay of cell adhesive activity.

### 2. Preparation of cell suspensions.

Subcultures of BHK cells were treated with PBS which contained 0.25% trypsin and 0.02% EDTA at 37°C for 2 minutes to detach the cells from the plate, and the cells were suspended in 10 ml of a 1:1 mixture of ice-cold Dulbecco's modified Eagle's (DME) medium and HEPES physiological saline (137 mM NaCl and 3 mM KCl in 20 mM HEPES buffer, pH 7.1). The suspension was centrifuged and the supernatant was discarded. The cells were suspended in 10 ml of an ice-cold mixture of DME medium and HEPES physiological saline containing 1 mg of soybean trypsin inhibitor (STI). The suspension was centrifuged and the precipitate was washed in an ice-cold mixture of DME and HEPES physiological saline which did not contain STI. These cells were suspended in the same mixture at the concentration of 5 x 10⁵ to 1 x 10⁶/ml.

### 3. Assay of cell adhesive activity.

In each well of the plate to which samples had been caused to adhere, 300 µl of the cell suspension was added, and the plate was incubated at 37°C for 1 hour. Then the wells were washed three times with a mixture of DME medium and HEPES at 37°C, and the non-adhering cells were removed. The remaining cells were fixed with a 4% formalin solution in PBS, and the shapes of the cells were examined by microscopy. The results are shown in Table 1.

**Table 1**

| Sample (5 µg/ml) | Cell adhesive activity | Calpastatin activity |
|---|---|---|
| HCSd42 | - | + |
| HCSd421 | - | + |
| HCSd342 | ++ | + |
| HCSd4210 | ++ | + |
| HCSd427 | ++ | + |
| HCSd4211 | ++ | + |

### [Effects of the invention]

As described above, this invention provides a method by which cell adhesive activity can be conferred to a polypeptide exhibiting calpain inhibition activity. With this invention, it is possible to enhance the affinity between the cells and the polypeptide, thereby enhancing the effect of drugs.

## Claims

1. An artificial polypeptide which, prior to modification, exhibits calpain inhibition activity and which, following intramolecular modification, exhibits both the original activity exhibited prior to modification and, in addition thereto, cell adhesive activity, wherein the modification is achieved by insertion of a polypeptide which contains at least the sequence RGD.

2. An artificial polypeptide according to claim 1 which has an amino acid sequence represented by the general following formula: 
KSLAPALPVESKPDKPSGKSGMDAALDDLIDTLGGPEETEEENTTYTGPE
VSDPMSSTYIEELGKREVTIPPKYRELLAKRGDSGPIGPDDAIDALSSDF
T(CGSPTAAGKKTEKEESTEVLKAQSAGTVRSAAPPQEK)a
 wherein a is either 1 or zero.

3. Genetic material, such as DNA, coding for a multifunctional polypeptide according to any preceding claim.

4. A method for the preparation by genetic engineering of a multifunctional polypeptide in which DNA coding for a peptide having cell adhesive activity is introduced into DNA coding for a peptide having calpain inhibition activity and a plasmid vector which carries the combined DNA is used to transform host cells so that they express a multifunctional polypeptide according to any preceding claim.

5. A method according to claim 4 in which the DNA coding for a peptide having cell adhesive activity comprises a nucleotide sequence coding for the amino acid sequence RGD.

6. A method according to claim 5 in which the said nucleotide sequence comprises CGHGGTGAC, wherein H symbolises A, C or T.

## Patentansprüche

1. Künstliches Polypeptid, welches vor der Modifikation eine Calpaininhibierende Aktivität aufweist und welches nach intramolekularer Modifikation sowohl die ursprüngliche vor der Modifikation gezeigte Aktivität als auch zusätzlich dazu eine Zelladhäsionsaktivität aufweist, wobei die Modifikation durch Insertion eines Polypeptids erreicht wird, welches mindestens die Sequenz RGD enthält.

2. Künstliches Polypeptid nach Anspruch 1, welches eine Aminosäuresequenz dargestellt durch die folgende allgemeine Formel 
KSLAPALPVESKPDKPSGKSGMDAALDDLIDTLGGPEETEEENTTYTGPE
VSDPMSSTYIEELGKREVTIPPKYRELLAKRGDSGPIGPDDAIDALSSDF
T(CGSPTAAGKKTEKEESTEVLKAQSAGTVRSAAPPQEK)a
 aufweist, wobei a entweder 1 oder 0 ist.

3. Genetisches Material, wie DNA, welches ein multifunktionales Polypeptid nach einem der vorhergehenden Ansprüche kodiert.

4. Verfahren zur Herstellung eines multifunktionalen Polypeptids mittels Gentechnologie, in welchem DNA, welche ein Peptid mit Zelladhäsionsaküvität kodiert, in DNA eingeführt wird, welche ein Peptid mit Calpain-inhibierender Aktivität kodiert, und ein Plasmidvektor, welcher die kombinierte DNA trägt, verwendet wird, um Wirtszellen zu transformieren, so daß sie ein multifunktionales Polypeptid nach einem der vorhergehenden Ansprüche exprimieren.

5. Verfahren nach Anspruch 4, in welchem die DNA, die ein Peptid mit Zelladhäsionsaktivität kodiert, eine Nukleotidsequenz umfaßt, welche die Aminosäuresequenz RGD kodiert.

6. Verfahren nach Anspruch 5, in welchem die Nukleotidsequenz CGHGGTGAC umfaßt, wobei H A, C oder T symbolisiert.

## Revendications

1. Polypeptide synthétique qui, avant modification, présente une activité inhibitrice de calpaïne et qui, après modification intramoléculaire, présente à la fois l'activité initiale présentée avant modification et, en plus de cette activité, une activité d'adhérence cellulaire, dans lequel la modification est effectuée par insertion d'un polypeptide qui contient au moins la séquence RGD.

2. Polypeptide synthétique suivant la revendication 1, qui a une séquence d'amino-acides représentée par la formule générale suivante : 
KSLAPALPVESKPDKPSGKSGMDAALDDLIDTLGGPEETEEENTTYTGPE
VSDPMSSTYIEELGKREVTIPPKYRELLAKRGDSGPIGPDDAIDALSSDF
T(CGSPTAAGKKTEKEESTEVLKAQSAGTVRSAAPPQEK)a
 dans laquelle a est égal à 1 ou 0.

3. Matériel génétique, tel qu'un ADN, codant pour un polypeptide multifonctionnel suivant l'une quelconque des revendications précédentes.

4. Procédé pour la préparation par génie génétique d'un polypeptide multifonctionnel dans lequel un ADN codant pour un peptide ayant une activité d'adhésivité cellulaire est introduit dans un ADN codant pour un peptide ayant une activité inhibitrice de calpaïne et un vecteur consistant en plasmide qui porte l'ADN combiné est utilisé pour transformer des cellules hôtes de telle sorte que ces cellules expriment un polypeptide multifonctionnel suivant l'une quelconque des revendications précédentes.

5. Procédé suivant la revendication 4, dans lequel l'ADN codant pour un peptide ayant une activité d'adhésivité cellulaire comprend une séquence de nucléotides codant pour la séquence d'amino-acides RGD.

6. Procédé suivant la revendication 5, dans lequel ladite séquence de nucléotides comprend la séquence CGHGGTGAC, dans laquelle H représente A, C ou T.
